# EUROPEAN PATENT APPLICATION

(11) **EP 3 301 100 A1**
(43) Date of publication of application: **04.04.2018**
(21) Application number: 16191930.3
(22) Date of filing: 30.09.2016
(51) Int. Cl.: C07F 17/02, A61P 33/00, A61K 31/295, A01N 37/00

(54) **ORGANOMETALLIC FLUCONAZOLE DERIVATES AND THEIR USE AS ANTIMYCOTICS**

(71) Applicant: Universität Zürich, 8006 Zürich (CH)
(72) Inventor: MASTROBUONI, Luclano, 8800 Thalwil (CH); GASSER, Gilles, F-75013 Paris (FR); RUBBLANI, Riccardo, D-78467 Konstanz (DE); HESS, Jeannine, CB2 1ND Cambridge (GB)
(74) Representative: Schulz Junghans Patentanwälte PartGmbB

(57) **Abstract**

The invention relates to compounds characterized by a general formula (1), 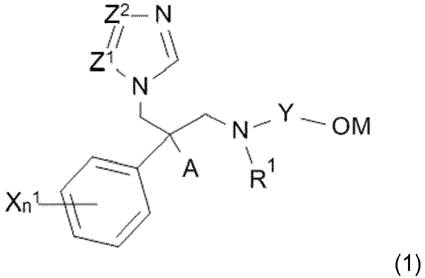 wherein OM is an organometallic compound. OM is independently selected from the group of an unsubstituted or substituted metal sandwich compound, an unsubstituted or substituted half metal sandwich compound or a metal carbonyl compound.

## Description

### Field of the invention

The present invention relates to fluconazole derivatives and their use as antimycotics.

### Background of the invention

Fungal infections are triggered by tiny parasites called *fungi*, present in water, soil, plants, invertebrates and mammalians. Their pathogen colonization can be superficial (cutaneous and subcutaneous level) or systemic. Local infections (e.g. *athlete's foot*) are contracted by 20-25% of the whole world population. Systemic parasitotis are the 4^{th}most common cause of bloodstream infections with a lifetime incidence rising up even to 75% (e.g. *candidiasis*)*.* Even if such dizzying morbidity still does not end up in a high mortality rate, local parasitosis together with invasive fungal infections (IFI) represent nowadays a grave problem, depressing the immune system, increasing the risk of contracting other pathologies and having indeed a deep impact on the whole health system economy. Although potent drugs are involved in antifungal therapy, the infections rate is warningly increasing every year. Moreover, many cases of developed resistance against conventional antimycotics appeared recently, especially for prolonged therapies (several weeks or months). Strangely, instead of observing a strong output in antifungal research, the discovery trend is not following the increment of disease cases. During the second half of 20^{th} century, antifungal-directed research mainly focused on an intensive derivatization of the already accepted compounds (e.g. ketoconazole → fluconazole, AmpB → Nystatin or AmpBlyposomes). Despite these efforts, just seven compounds are nowadays on the list of World Health Organization Essential Medicine (WHO-EM, data of October 2013). Moreover, the benchmark drugs were all discovered almost 20 or more years ago. Novel strategies to fight fungal infections are therefore urgently required.

The drugs Salvarsan (1909 against syphilis) and Cisplatin (1965-1978 against cancer) are undoubtedly the best examples of metal-based drugs which have reached the market. Other Platinum derivatives such as Oxaliplatin and Carboplatin are nowadays involved in chemotherapeutic treatments against several cancers including ovarian, lung and colon carcinoma. Other metal-based compounds containing technetium or gadolinium are also used in the clinics worldwide as imaging agents. Gold complexes are benchmark products against rheumatoid arthritis. An organometallic derivative of the well-known antimalarial drug Chloroquine, namely Ferroquine, has shown extremely promising results as a novel antimalarial drug candidate. These stunning examples of the use of metal complexes contrasts with the relatively low efforts made in the utilization of such compounds in the field of antifungal therapy. Sporadic reports proved the effectiveness of different metals (e.g. Co(II), Ni(II), Cu(II), Pd(ll)) bearing a variety of ligands (e.g. azole moieties, thiosemicarbazones, carboxamides) against several fungal strains.

The two most common antifungal drugs, inscribed in the List of Essential Medicines of the WHO and commercially available, are Clotrimazole and Fluconazole. Both these therapeutics are belonging to the azole class. Fluconazole can be applied topically as well as systemically and is used predominantly for the treatment of Candidiasis. Both those azoles are specifically targeting the enzyme lanosterol-14α-demethylase which plays a central role in the biosynthesis of ergosterol, an important fungal wall component. The inhibition of this protein leads to wall instability and fungal death. Mammalian cells use cholesterol instead of ergosterol which reduces unwanted side effects by inhibition of this enzyme.

The widespread use of Clotrimazole as well as Fluconazole against skin, genital and invasive fungal infections, has given rise to fungal strains with a resistance against these substances. This limits the usability of Clotrimazole and Fluconazole especially in the treatment of Candidiasis.

Based on the above mentioned state of the art, the objective of the present invention is to provide means and methods to use organometallic compounds as antifungal agents, in particular agents able to overcome a resistance against known antifungal agent. This objective is attained by the claims of the present specification.

### Summary of the invention

The inventors unexpectedly discovered that the potency of derivatives of Fluconazole comprising an organometallic compound as antifungal agent is vastly increased. Even fungal strains resistant to treatment with Fluconazole can be effectively treated by the compounds of the present invention.

The present invention discloses the organometallic derivatization of Fluconazole with a metallocenyl and in particular a ferrocenyl moiety at the C3 position. The azole group (imidazole, triazole or tetrazole), which is carrying the inhibition activity, and the mono- or disubstituted phenyl group responsible for active site interaction. Without wishing to be bound by theory the metallocenyl derivatization is supposed to play an important role for the overall biodistribution and uptake (increasing the lipophilicity of the parent drug). Moreover, the metal center, in particular the iron center, possesses redox catalytic properties that could alter the antioxidant balance of the target microorganism, enhancing the overall activity with a second additional mode of action.

According to a first aspect of the invention provided herein are organometallic compounds characterized by a general formula (1), 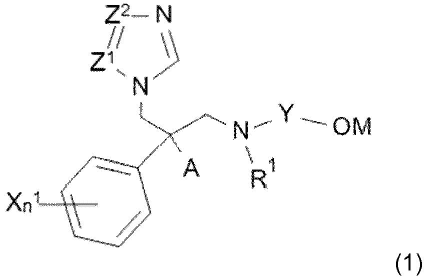 or a salt thereof wherein
- Z¹ and Z² are independently from each other -C or -N,
- A is selected from -H, -OH, -SH, a substituted or unsubstituted C₁ to C₄ thiol, a substituted or unsubstituted C₁ to C₄ alkylamine, a substituted or unsubstituted C₁ to C₄ alkoxy, a substituted or unsubstituted C₁ to C₄ aldehyde, a substituted or unsubstituted C₃-C₆ heterocycle,
- R¹ is hydrogen, a substituted or unsubstituted C₁ to C₅ alkyl, substituted or unsubstituted C₃-C₈ cycloalkyl, substituted or unsubstituted C₁ to C₅ alkenyl, substituted or unsubstituted C₁ to C₅ alkynyl,
- Y is a substituted or unsubstituted C₁ to C₅ alkyl, substituted or unsubstituted C₃-C₈ cycloalkyl, substituted or unsubstituted C₁ to C₅ alkenyl, substituted or unsubstituted C₁ to C₅ alkynyl, -OR^{a}-, -C(O)R^{a}-, -C(S)OR^{a}-, -R^{a}-O-R^{a}-, -R^{a}-CO(=O)-R^{a}-,
   wherein each R^{a} is independently from each other R^{a} selected from the group consisting of hydrogen, unsubstituted C₁ to C₄ alkyl, and C₁ to C₄ alkyl substituted with C₁ to C₄ alkoxy,
- OM is an organometallic compound independently selected from the group of an unsubstituted or substituted metal sandwich compound, an unsubstituted or substituted half metal sandwich compound or a metal carbonyl compound,
- n of Xₙ¹ is 1 or 2 and each X¹ independently from each other X¹ is -C(=O)OR², -C(=O)NR²₂, -C(=O)SR², -C(=S)OR², -C(NH)NR²₂, -CN₄H₂, -NR²₂, -C(=O)R², -C(=S)R², -OR², -SR², -CF₃,-OCF₃, -SCF₃, -SOCF₃, -SO₂CF₃, -CN, -NO₂, -F, -Cl, -Br or -I, with each R² independently from any other R² being a hydrogen or C₁₋C₄ alkyl.

Salts of the compound of general formula (1) include all halides, in particular chloride, bromide, iodide, fluoride, more particular chloride and bromide.

The term "substituted" refers to the addition of a substituent group to a parent compound.

"Substituent groups" can be protected or unprotected and can be added to one available site or to many available sites in a parent compound. Substituent groups may also be further substituted with other substituent groups and may be attached directly or by a linking group such as an alkyl or hydrocarbyl group to a parent compound. "Substituent groups" amenable herein include, without limitation, halogen, oxygen, nitrogen, sulphur, hydroxyl, alkyl, alkenyl, alkynyl, acyl (-C(O)R^{α}), carboxyl (-C(O)OR^{α}), aliphatic groups, alicyclic groups, alkoxy, substituted oxy (-OR^{α}), aryl, aralkyl, heterocyclic radical, heteroaryl, heteroarylalkyl, amino (-N(R^{β})(R^{γ})), imino(=NR^{β}), amido (-C(O)N(R^{β})(R^{γ}) or -N(R^{β})C(O)R^{α}), hydrazine derivates (-C(NH)NR^{α}R^{β}), tetrazole(CN₄H₂), azido (-N₃), nitro (-NO₂), cyano (-CN), isocyano (-NC), cyanato (-OCN), isocyanato (-NCO), thiocyanato (-SCN); isothiocyanato (-NCS); carbamido (-OC(O)N(R^{β})(R^{γ}) or -N(R^{β})C(O)OR^{α}), thiol (-SR^{β}), sulfinyl (-S(O)R^{β}), sulfonyl (-S(O)₂R**)**^{β}, sulfonamidyl (-S(O)₂N(R^{β})(R^{γ})or -N(R^{β})S(O)₂R^{β}) and fluorinated compounds -CF₃, -OCF₃, -SCF₃, -SOCF₃ or -SO₂CF₃. Wherein each R^{α}, R^{β} and R^{γ} is, independently, H or a further substituent group with a preferred list including without limitation, H, alkyl, alkenyl, alkynyl, aliphatic, alkoxy, acyl, aryl, heteroaryl, alicyclic, heterocyclic and heteroarylalkyl.

As used herein the term "alkyl" refers to a saturated straight or branched hydrocarbon moiety containing up to 10, particularly up to 5 carbon atoms. Examples of alkyl groups include, without limitation, methyl, ethyl, propyl, butyl, isopropyl, n-hexyl, octyl, decyl and the like. Alkyl groups typically include from 1 to about 10 carbon atoms (C₁-C₁₀ alkyl), particularly with from 1 to about 5 carbon atoms (C₁-C₅ alkyl). The term "cycloalkyl" refers to an interconnected alkyl group forming a ring structure. Alkyl or cycloalkyl groups as used herein may optionally include further substituent groups.

As used herein the term "alkenyl" refers to a straight or branched hydrocarbon chain moiety containing up to 10 carbon atoms and having at least one carbon-carbon double bond. Examples of alkenyl groups include, without limitation, ethenyl, propenyl, butenyl, 1-methyl-2-buten-1-yl, dienes such as 1,3-butadiene and the like. Alkenyl groups typically include from 2 to about 10 carbon atoms, more typically from 2 to about 4 carbon atoms. Alkenyl groups as used herein may optionally include further substituent groups.

As used herein the term "alkynyl" refers to a straight or branched hydrocarbon moiety containing up to 10 carbon atoms and having at least one carbon-carbon triple bond. Examples of alkynyl groups include, without limitation, ethynyl, 1-propynyl, 1-butynyl, and the like. Alkynyl groups typically include from 2 to about 10 carbon atoms, more typically from 2 to about 4 carbon atoms. Alkynyl groups as used herein may optionally include further substituent groups.

As used herein the term "alkoxy" refers to an oxygen-alkyl moiety, wherein the oxygen atom is used to attach the alkoxy group to a parent molecule. Examples of alkoxy groups include without limitation, methoxy, ethoxy, propoxy, isopropoxy, n-butoxy, sec-butoxy, *tert*-butoxy, n-pentoxy, neopentoxy, n-hexoxy and the like. The term "cycloalkoxy" refers to an interconnected alkoxy group forming a ring structure. Alkoxy or cycloalkoxy groups as used herein may optionally include further substituent groups.

As used herein the term "aryl" refers to a hydrocarbon with alternating double and single bonds between the carbon atoms forming a ring structure (in the following an "aromatic hydrocarbon"). The term "heteroaryl" refers to aryl compounds in which at least one carbon atom is replaced with an oxygen, a nitrogen or a sulphur atom. The aromatic hydrocarbon may be neutral or charged. Examples of aryl or heteroaryl groups are benzene, pyridine, pyrrole or cyclopenta-1,3-diene-anion. Ary lor heteroaryl groups as used herein may optionally include further substituent groups.

As used herein the term "organometallic compound" refers to a compound comprising a metal, in particular a transition metal (a metal selected from the group 3 to group 12 metals of the periodic table), bound to at least one carbon.

As used herein the term "metal sandwich compound" refers to a compound comprising a metal, in particular a transition metal, bound to two aryl or heteroaryl ligands (in the following "sandwich ligands") by a haptic covalent bound. The aryl or heteroaryl ligands may be unsubstituted or substituted.

As used herein the term "half metal sandwich compound" refers to a compound comprising a metal, in particular a transition metal, bound to just one aryl or heteroaryl ligand (sandwich ligand). The other ligand may comprise - without being limited to - alkyl, allyl, CN or CO, in particular CO.

As used herein the term "metal carbonyl compound" refers to a coordination complex of at least one transition metal with a carbon monoxide (CO) ligand. It may comprise a neutral, anionic or cationic complex. The carbon monoxide ligand may be bound terminally to a single metal atom or may be bridging to two or more metal atoms. The complex may be homoeleptic (containing only carbon monoxide ligands) or heteroeleptic.

As used herein the term "metallocene" refers to a metal sandwich compound comprising an aryl or heteroaryl five ring ligand (in the following "cp-ligand" or "hetero cp-ligand").

In some embodiments the upper ring structure in general formula (1) may be an imidazole with Z¹ and Z² being a -C. In some embodiments the upper ring structure may be a triazole with Z¹ being a -N and Z² being a -C. In some embodiments the upper ring structure may be a tetrazole with Z¹ and Z² being a -N.

In some embodiments the organometallic compound (OM) may be attached to the -C-N(R¹)-moiety of the parent compound by a linker comprising a methyl with Y being a C₁-alkyl. In some embodiment the OM may be attached by a linker comprising a C₂ to C₅ alkyl. In some embodiments the OM may be attached by a linker comprising a C₃ to C₈ cycloalkyl, in particular with the OM being in ortho or para position to the attachment point of the linker with the parent compound.

In some embodiments the n of Xₙ¹ is 1 or 2, and each X¹ independently from any other X¹ is -CN, -CF₃, -OCF₃, -SCF₃, -SOCF₃, -SO₂CF₃,-F, -Cl, -Br or -I, in particular each X¹ independently from any other X¹ is -F, -Cl, -Br or -I.

In some embodiments, n of Xₙ¹ is 2 and one of the two X¹ is in ortho position and the other X¹ is in para position to the attachment position of the benzene moiety.

In some embodiments, n of Xₙ¹ is 2 and one of the two X¹ is in meta position and the other X¹ is in para position to the attachment position of the benzene moiety.

In some embodiments, the amine moiety of the parent compound binding the linker Y is a secondary amine with R¹ being a hydrogen. In some embodiments the amine moiety of the parent compound binding the linker Y is a tertiary amine with R¹ being a substituted or unsubstituted C₁ to C₃ alkyl.

In some embodiments, the organometallic compound (OM) is separated from the parent compound by a linker with Y being a substituted or unsubstituted C₁ to C₃ alkyl.

In some embodiments, OM is a metal sandwich complex, wherein each of the two sandwich ligands is selected independently from a five-membered or six-membered aryl group or a five-membered or six-membered heteroaryl group. In some embodiments, OM is a metal sandwich complex, wherein both sandwich ligands are the same and are selected from a five-membered or six-membered aryl group or a five-membered or six-membered heteroaryl group. In some embodiments, OM is a metal sandwich complex, wherein at least one of the two ligands is selected from a five-membered or six-membered aryl group, wherein the other is selected from a five-membered or six-membered heteroaryl group. In some embodiments, OM is a substituted or unsubstituted metallocene, wherein each of two ligands is selected independently from a five-membered aryl group (cp-ligand) or a five-membered heteroaryl group (hetero cp-ligand). The metal sandwich complex may be connected to the parent molecule by any atom of one of the two sandwich ligands.

In some embodiments, the organometallic compound is characterized by a general formula (2a) 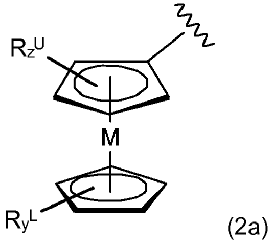 wherein M is a metal selected from Fe, Ru, Co, Ni, Cr, Os or Mn, and z of R_{z}^{U} is 0, 1, 2, 3 or 4, and y of Ry^{L} is 0, 1, 2, 3, 4 or 5 and each R^{L} and each R^{U} are independently from any other R^{L} and R^{U} selected from
- an unsubstituted or substituted C₁-C₁₀ alkyl, an unsubstituted or substituted C₂-C₁₀ alkenyl, an unsubstituted or substituted C₂-C₁₀ alkynyl, an unsubstituted or substituted C₃-C₈ cycloalkyl, an unsubstituted or substituted C₁-C₁₀ alkoxy, an unsubstituted or substituted C₃-C₈ cycloalkoxy,
- an unsubstituted or substituted C₆-C₁₄ aryl,
- an unsubstituted or substituted 5- to 10-membered heteroaryl, wherein 1 to 4 ring atoms are independently selected from nitrogen, oxygen or sulfur,
- an unsubstituted or substituted 5- to 10-membered heteroalicyclic ring, wherein 1 to 3 ring atoms are independently nitrogen, oxygen or sulfur,
- -OR³, -SR³, -C(O)R³,-C(S)R³ -C(O)OR³,-C(S)OR³, -C(O)SR³, -C(O)NR³R⁴, -NR³R⁴, -S(O)₂R³, -S(O)₂OR³ and -S(O)₂NR³R⁴,
   wherein
   R³ and R⁴ are independently selected from the group consisting of hydrogen, unsubstituted C₁-C₄ alkyl, and C₁-C₄ alkyl substituted with C₁-C₄ alkoxy,
- general formula (3a) or (3b)

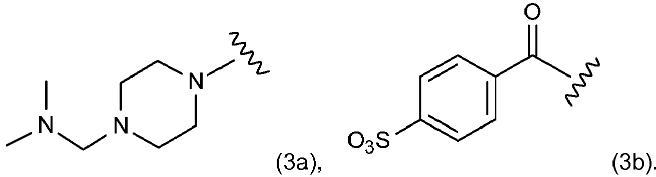

In some embodiments, the upper of the sandwich ligands is substituted by a compound according to general formula (3a) with z of R_{z}^{U} being 1 and R^{U} being general formula (3a). In some embodiments, the upper of the sandwich ligands is substituted by a compound according to general formula (3b) with z of R_{z}^{U} being 1 and R^{U} being general formula (3b).

In some embodiments, the lower of the sandwich ligands is substituted by a compound according to general formula (3a) with y of Ry^{L} being 1 and R^{L} being general formula (3a). In some embodiments, the lower of the sandwich ligands is substituted by a compound according to general formula (3b) with y of Ry^{L} being 1 and R^{L} being general formula (3b).

In some embodiments, OM is unsubstituted ferrocene with M of general formula (2a) being Fe and y and z being 0.

In some embodiments, OM is a substituted ferrocene with M of general formula (2a) being Fe and z of R_{z}^{U} is 0, 1, 2, 3 or 4, and y of Ry^{L} is 0, 1, 2, 3, 4 or 5 and
each R^{L} and each R^{U} are independently from any other R^{L} and R^{U}selected from
- an unsubstituted or substituted C₁-C₁₀ alkyl, an unsubstituted or substituted C₂-C₁₀ alkenyl, an unsubstituted or substituted C₂-C₁₀ alkynyl, an unsubstituted or substituted C₃-C₈ cycloalkyl, an unsubstituted or substituted C₁-C₁₀ alkoxy, an unsubstituted or substituted C₃-C₈ cycloalkoxy,
- an unsubstituted or substituted C₆-C₁₄ aryl,
- an unsubstituted or substituted 5- to 10-membered heteroaryl, wherein 1 to 4 ring atoms are independently selected from nitrogen, oxygen or sulfur,
- an unsubstituted or substituted 5- to 10-membered heteroalicyclic ring, wherein 1 to 3 ring atoms are independently nitrogen, oxygen or sulfur,
- -OR³, -SR³, -C(O)R³, -C(S)R³, -C(O)OR³, -C(S)OR³, -C(O)SR³, -C(O)NR³R⁴, -NR³R⁴, -S(O)₂R³, -S(O)₂OR³ and -S(O)₂NR³R⁴,
   wherein
   R³ and R⁴ are independently selected from the group consisting of hydrogen, unsubstituted C₁-C₄ alkyl, and C₁-C₄ alkyl substituted with C₁-C₄ alkoxy,
- general formula (3a) or (3b).

In some embodiments, OM is a substituted ferrocene with M of general formula (2a) being Fe and y of Ry^{L} being 1 with R^{L} being general formula (3a). In some embodiments, OM is a substituted ferrocene with M of general formula (2a) being Fe and y of Ry^{L} being 1 with R^{L} being general formula (3b).

In some embodiments, OM is a substituted ferrocene with M of general formula (2a) being Fe and z of R_{z}^{U} being 1 with R^{U} being general formula (3a). In some embodiments, OM is a substituted ferrocene with M of general formula (2a) being Fe and z of R_{z}^{U} being 1 with R^{U} being general formula (3b).

In some embodiments, each R^{L} and each R^{U} is independently from any other R^{L} and R^{U}selected from -OR³, -SR³, -C(O)R³, -C(S)R³, -C(O)OR³, -C(S)OR³, -C(O)SR³, -C(O)NR³R⁴, -NR³R⁴, -S(O)2 R³, -S(O)₂O R³, and -S(O)₂NR³R⁴, wherein
R³ and R⁴ are independently selected from the group consisting of hydrogen, unsubstituted C₁-C₄ alkyl, and C₁-C₄ alkyl substituted with C₁-C₄ alkoxy.

In some embodiments, each R^{L} and each R^{U} is independently from any other R^{L} and R^{U} selected from general formula (3a).

In some embodiments, each R^{L} and each R^{U} is independently from any other R^{L} and R^{U} selected from general formula (3b).

In some embodiments, OM is a metallocene with M being selected from Fe, Ru or Co.

In some embodiments, OM is an unsubstituted metallocene with M being selected from Fe, Ru or Co and y and z being 0.

In some embodiments, OM is an organometallic compound according to the general formula (2b), 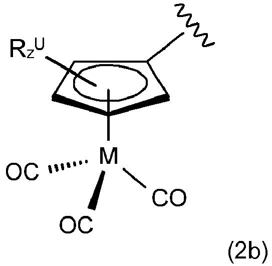
wherein M is a metal selected from the group of Mn, Re or Tc, and
z of R_{z}^{U} is 0,1,2,3 or 4, and
each R^{U} is independently from any other R^{U} selected from
- an unsubstituted or substituted C₁-C₁₀ alkyl, an unsubstituted or substituted C₂-C₁₀ alkenyl, an unsubstituted or substituted C₂-C₁₀ alkynyl, an unsubstituted or substituted C₃-C₈ cycloalkyl, an unsubstituted or substituted C₁-C₁₀ alkoxy, an unsubstituted or substituted C₃-C₈ cycloalkoxy,
- an unsubstituted or substituted C₆-C₁₄ aryl,
- an unsubstituted or substituted 5- to 10-membered heteroaryl, wherein 1 to 4 ring atoms are independently selected from nitrogen, oxygen or sulfur,
- an unsubstituted or substituted 5- to 10-membered heteroalicyclic ring, wherein 1 to 3 ring atoms are independently nitrogen, oxygen or sulfur,
- -OR³, -SR³, -C(O)R³, -C(S)R³, -C(O)OR³, -C(S)OR³, -C(O)SR³, -C(O)NR³R⁴, -NR³R⁴, -S(O)₂R³, -S(O)₂OR³, and -S(O)₂NR³R⁴,
   wherein
   R³ and R⁴ are independently selected from the group consisting of hydrogen, unsubstituted C₁-C₄ alkyl, and C₁-C₄ alkyl substituted with C₁-C₄ alkoxy.

In some embodiments, z of R_{z}^{U} of the general formula (2b) is 0, 1, 2, 3 or 4, and each R^{U} is independently from any other R^{U} selected from -OR³, -SR³, -C(O)R³, -C(S)R³, -C(O)OR³, -C(S)OR³, -C(O)SR³, -C(O)NR³R⁴, -NR³R⁴, -S(O)₂R³, -S(O)₂OR³, and -S(O)₂NR³R⁴, wherein R³ and R⁴ are independently selected from the group consisting of hydrogen, unsubstituted C₁-C₄ alkyl, and C₁-C₄ alkyl substituted with C₁-C₄ alkoxy.

In some embodiments, z of R_{z}^{U} of the general formula (2b) is 1, and R^{U} is selected from -OR³, -SR³, -C(O)R³, -C(S)R³, -C(O)OR³, -C(S)OR³, -C(O)SR³, -C(O)NR³R⁴, -NR³R⁴, -S(O)₂R³, -S(O)₂OR³ and -S(O)₂NR³R⁴, wherein R³ and R⁴ are independently selected from the group consisting of hydrogen, unsubstituted C₁-C₄ alkyl, and C₁-C₄ alkyl substituted with C₁-C₄ alkoxy.

In some embodiments, z of R_{z}^{U} of the general formula 2b is 0.

In some embodiments, OM is an organometallic compound according to the general formula (2c), 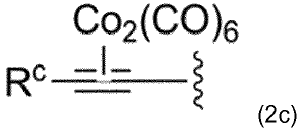
wherein R^{c} is selected from
   - hydrogen,
   - an unsubstituted or substituted C₁-C₁₀ alkyl, an unsubstituted or substituted C₂-C₁₀ alkenyl, an unsubstituted or substituted C₂-C₁₀ alkynyl, an unsubstituted or substituted C₃-C₈ cycloalkyl, an unsubstituted or substituted C₁-C₁₀ alkoxy, an unsubstituted or substituted C₃-C₈ cycloalkoxy,
   - an unsubstituted or substituted C₆-C₁₄ aryl,
   - an unsubstituted or substituted 5- to 10-membered heteroaryl, wherein 1 to 4 ring atoms are independently selected from nitrogen, oxygen or sulfur,
   - an unsubstituted or substituted 5- to 10-membered heteroalicyclic ring, wherein 1 to 3 ring atoms are independently nitrogen, oxygen or sulfur,
   - -OR³, -SR³, -C(O)R³, -C(S)R³, -C(O)OR³, -C(S)OR³, -C(O)SR³, -C(O)NR³R⁴, -NR³R⁴, -S(O)₂R³, -S(O)₂OR³, and -S(O)₂NR³R⁴,
      wherein
      R³ and R⁴ are independently selected from the group consisting of hydrogen, unsubstituted C₁-C₄ alkyl, and C₁-C₄ alkyl substituted with C₁-C₄ alkoxy.

In some embodiments, R^{c} of the general formula (2c) is selected from -OR³, -SR³, -C(O)R³, -C(S)R³, -C(O)OR³, -C(S)OR³, -C(O)SR³, -C(O)NR³R⁴, -NR³R⁴, -S(O)₂R³, -S(O)₂OR³, and -S(O)₂NR³R⁴, wherein R³ and R⁴ are independently selected from the group consisting of hydrogen, unsubstituted C₁-C₄ alkyl, and C₁-C₄ alkyl substituted with C₁-C₄ alkoxy.

In some embodiments, R^{c} of the general formula (2c) is hydrogen.

In some embodiments, R^{c} of the general formula (2c) is an unsubstituted or substituted C₁-C₁₀ alkyl, an unsubstituted or substituted C₁-C₁₀ alkenyl, an unsubstituted or substituted C₁-C₁₀ alkynyl, an unsubstituted or substituted C₃-C₈ cycloalkyl, an unsubstituted or substituted C₁-C₁₀ alkoxy, an unsubstituted or substituted C₃-C₈ cycloalkoxy.

Particular embodiments of this aspect of the invention are:
a. syn/anti-N-(ferrocenylmethyl)-2-(2,4-difluorophenyl)-2-hydroxy-3-(1H-1,2,4-triazol-1-yl)propan-1-amine, compound 4 with Z¹=N, Z²=C, A=OH, R¹=hydrogen, Y=methyl, OM= unsubstituted ferrocenyl and n of Xₙ¹ is 2 and each X¹ is F: 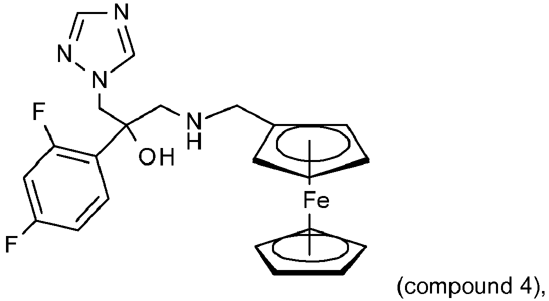
b. syn/anti-N-(ferrocenylmethyl)-2-(2,4-difluorophenyl)-2-hydroxy-3-(1H-1,2,4-triazol-1-yl)propan-1-aminium chloride, compound 4a with Z¹=N, Z²=C, A=OH, R¹=hydrogen, Y=methyl, OM= unsubstituted ferrocenyl and n of Xₙ¹ is 2 and each X¹ is F: 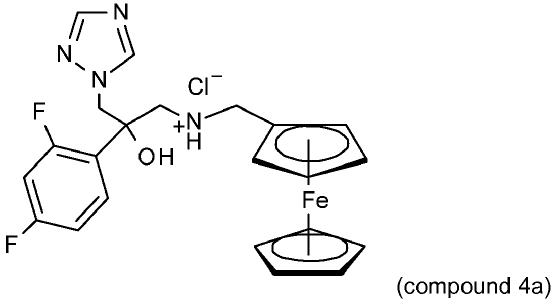
c. syn/anti-N-(ferrocenylmethyl)-2-(2,4-difluorophenyl)-2-hydroxy-N-methyl-3-(1H-1,2,4-triazol-1-yl)propan-1-amine, compound 5 with Z¹=N, Z²=C, A=OH, R¹=methyl, Y=methyl, OM= unsubstituted ferrocenyl and n of Xₙ¹ is 2 and each X¹ is F 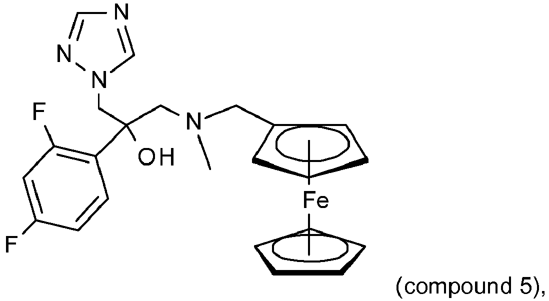
d. syn/anti-N-(ferrocenylmethyl)-2-(2,4-difluorophenyl)-2-hydroxy-N-methyl-3-(1H-1,2,4-triazol-1-yl)propan-1-aminium chloride, compound 5a with Z¹=N, Z²=C, A=OH, R¹=methyl, Y=methyl, OM= unsubstituted ferrocenyl and n of Xₙ¹ is 2 and each X¹ is F 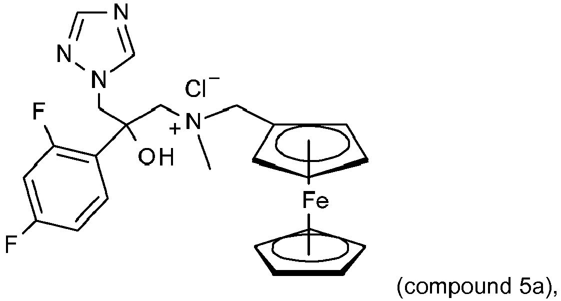
e. syn/anti-N-(ferrocenylmethyl)-2-(2,4-difluorophenyl)-2-hydroxy-N-ethyl-3-(1H-1,2,4-triazol-1-yl)propan-1-amine, compound 6 with Z¹=N, Z²=C, A=OH, R¹=ethyl, Y=methyl, OM= unsubstituted ferrocenyl and n of Xₙ¹ is 2 and each X¹ is F 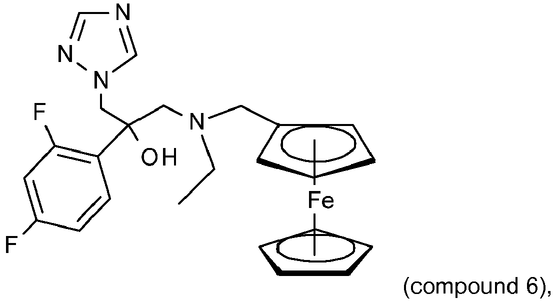
f. syn/anti-N-(ferrocenylmethyl)-2-(2,4-difluorophenyl)-2-hydroxy-N-ethyl-3-(1H-1,2,4-triazol-1-yl)propan-1-aminium chloride, compound 6a with Z¹=N, Z²=C, A=OH, R¹=ethyl, Y=methyl, OM= unsubstituted ferrocenyl and n of Xₙ¹ is 2 and each X¹ is F 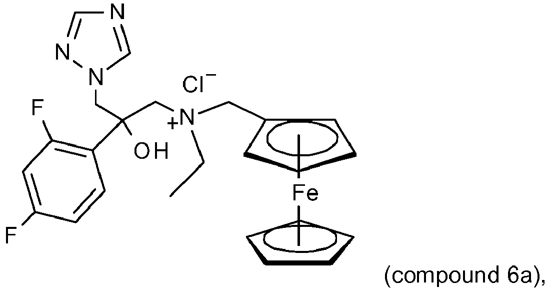
g. syn/anti-N-(ferrocenylmethyl)-2-(2,4-difluorophenyl)-2-hydroxy-N-isopropyl-3-(1H-1,2,4-triazol-1-yl)propan-1-amine, compound 7 with Z¹=N, Z²=C, A=OH, R¹=isopropyl, Y=methyl, OM= unsubstituted ferrocenyl and n of Xₙ¹ is 2 and each X¹ is F 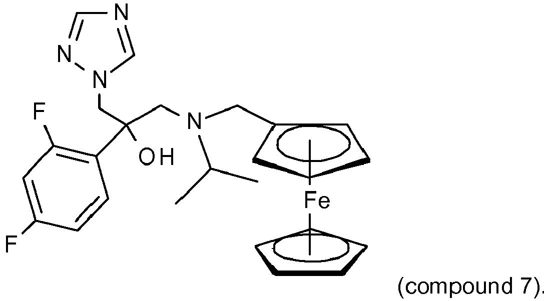
h. syn/anti-N-(ferrocenylmethyl)-2-(2,4-difluorophenyl)-2-hydroxy-N-isopropyl-3-(1H-1,2,4-triazol-1-yl)propan-1-aminium chloride, compound 7a with Z¹=N, Z²=C, A=OH, R¹=isopropyl, Y=methyl, OM= unsubstituted ferrocenyl and n of Xₙ¹ is 2 and each X¹ is F

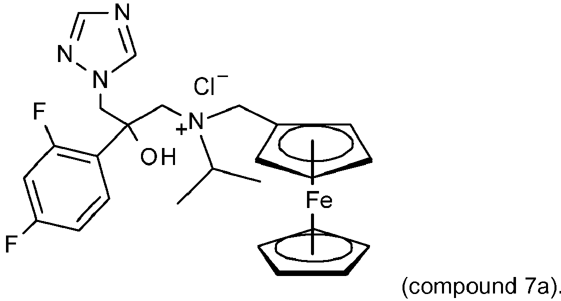

The compounds of the general formula (1) can also be obtained in the form of their hydrates and/or also can include other solvents used for example for the crystallization of compounds present in the solid form. Depending on the method and/or the reaction conditions, compounds of the general formula (1) can be obtained in the free form or in the form of salts.

The compounds of the general formula (1) may be present as optical isomers or as mixtures thereof. The stereocenter in the formulas is located on the C₁ carbon atom of the ethyl moiety. The invention relates both to the pure isomers, racemic mixtures and all possible isomeric mixtures and is hereinafter understood as doing so, even if stereochemical details are not specifically mentioned in every case. Diastereoisomeric mixtures of compounds of the general formula (1), which are obtainable by the process or any other way, may be separated in known manner - on the basis of the physical-chemical differences of their components - into pure diastereoisomers, for example by fractional crystallisation, distillation and/or chromatography, in particular by preparative HPLC using a chiral HPLC column.

If not stated otherwise a racemic mixture is used.

According to the invention, apart from separation of corresponding isomer mixtures, generally known methods of diastereoselective or enantioselective synthesis can also be applied to obtain pure diastereoisomers or enantiomers, e.g. by carrying out the method described hereinafter and using educts with correspondingly suitable stereochemistry.

It is advantageous to isolate or synthesise the biologically more active isomer, provided that the individual compounds have different biological activities.

A further aspect of the invention is the process for the preparation of the compounds described by the general formula (1).

The preparation comprises a compound (9) described by the formula 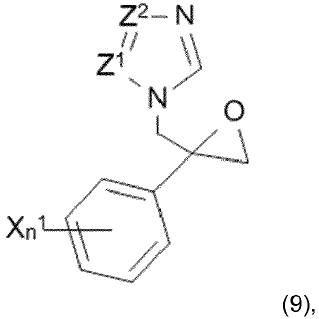 with Xₙ¹, Z¹ and Z² having the same meaning as defined above. The compound is synthesized by a Corey-Chaykovski epoxidation. The reaction pathway is depicted in scheme 1.

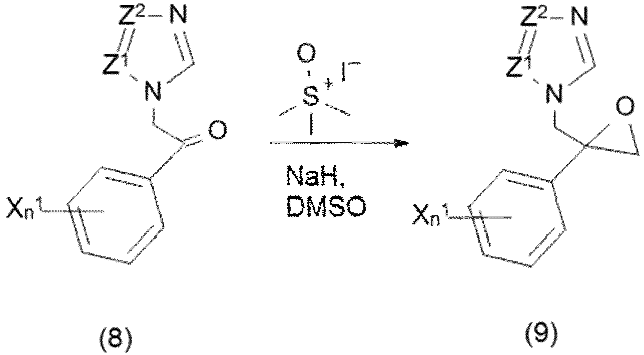

Scheme 1: Xₙ¹ Z¹ and Z² have the same meaning as defined above. Compound (8) includes the commercially available 2,4-difluoro-a-(1H-1,2,4-triazolyl)acetophenone (CAS number 86404-63-9) in the case of n=2 and X¹=F , Z¹=C and Z²=N and was reacted by Corey-Chaykovski epoxidation to compound (9).

In one embodiment, compound (9) was treated with compound (10; N-alkyl-methylaminoferrocene) yielding compound (11) by nucleophilic addition. The reaction pathway is depicted in scheme 2.

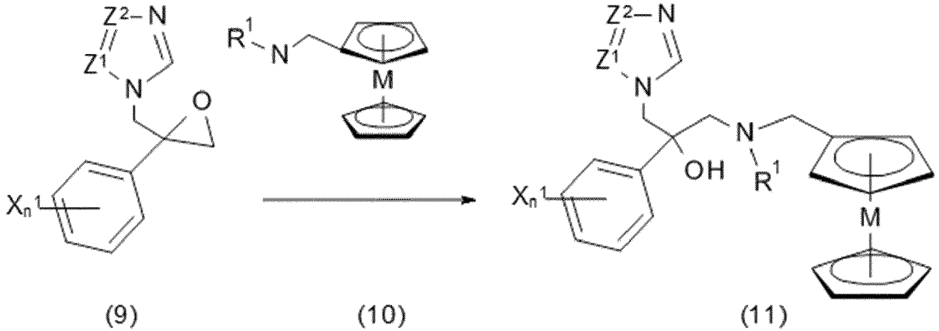

Scheme 2: Compound (10) was reacted via nucleophilic addition with the epoxide ring compound (9) yielding compound (11). Xₙ¹, Z¹, Z², M and R¹ have the same meaning as defined above. The reaction scheme is applicable for the synthesis of compounds (4), (5), (6) and (7) in case of n=2 and X¹=F, M=Fe, Z¹=C and Z²=N and R¹ being H, C₁, C₂- or C₃-alkyl, respectively. Compound (10) may also include different linkers Y. The given reaction scheme with Y=methyl is usable for the other linkers Y mentioned above.

Reaction pathways for compounds comprising the half metal sandwich complexes OM of the general formula (2b) follow a similar pathway as the above mentioned reactions, in particular a similar pathway as depicted in scheme 2, which is easily adaptable for a person skilled in the art. Reference is made to the cited conditions, references and reactions pathways. Reaction pathways for compounds comprising the cobalt carbonyl complex OM of the general formula (2c) follow a similar pathways as the above mentioned reactions in particular a similar pathway as depicted in scheme 2, which is easily adaptable for a person skilled in the art. In particular starting with an alkinyl that is coupled to a cobalt carbonyl resulting in a organometallic compound according to general formula (2c) that is coupled to the parent compound as described in Gasser et al. (Inorg. Chem. 2009, 48, 3157-3166).

According to a second aspect of the invention, the compounds defined in the first aspect of the invention are provided for the use as a medicament.

According to a third aspect of the invention, the compounds defined in the first aspect of the invention are provided for the use as an antimycotic substance. In particular the compounds provided are for treating infections caused by the Ascomycota and Basidiomycota famlies in particular species of *Candida albicans*, *Candida glabrata*, *other Candidiasis*, *Aspergillus fumigatus, Aspergillus flavus, Cryptococcus neoformans, Cryptococcus gattii, Histoplasma capsulatum, Pneumocystis jirovecii, Tinea pedis, Tinea corporis, Blastommycesdermatidis.* The method is comprising the administration of a compound according to the above aspects or embodiments of the invention to a patient in need thereof.

The treatment may be for prophylactic or therapeutic purposes. For administration, a compound according to the above aspect of the invention is preferably provided in the form of a pharmaceutical preparation comprising the compound in chemically pure form and optionally a pharmaceutically acceptable carrier and optionally adjuvants. The compound is used in an amount effective against antifungal infection. The dosage of the compound depends upon the species, the patient age, weight, and individual condition, the individual pharmacokinetic data, mode of administration, and whether the administration is for prophylactic or therapeutic purposes. The daily dose administered ranges from approximately 1 µg/kg to approximately 1000 mg/kg, preferably from approximately 1µg to approximately 100 µg, of the active agent according to the invention.

According to a fourth aspect of the invention, the compounds defined in the first aspect of the invention are provided for the use in the treatment of fungal infections caused by azole-resistant *Candida albicans.*

Pharmaceutically acceptable salts of the compounds provided herein are deemed to be encompassed by the scope of the present invention.

According to one aspect of the invention, a pharmaceutical composition for preventing or treating fungal infection, particularly infection by the Ascomycota and Basidiomycota famlies in particular species of *Candida albicans*, *Candida glabrata*, *other Candidiasis*, *Aspergillus fumigatus, Aspergillus flavus*, *Cryptococcus neoformans, Cryptococcus gattii, Histoplasma capsulatum*, *Pneumocystis jirovecii*, *Tinea pedis*, *Tinea corporis*, *Blastommycesdermatidis* is provided, comprising a compound according to the above aspects or embodiments of the invention.

The pharmaceutical compositions comprise approximately 1% to approximately 95% active ingredient, preferably from approximately 20% to approximately 90% active ingredient. According to one aspect of the invention, a dosage form for preventing or treating antifungal infection, particularly infection by by the Ascomycota and Basidiomycota famlies in particular species of *Candida albicans*, *Candida glabrata*, *other Candidiasis*, *Aspergillus fumigatus*, *Aspergillus flavus, Cryptococcus neoformans, Cryptococcus gattii, Histoplasma capsulatum*, *Pneumocystis jirovecii*, *Tinea pedis*, *Tinea corporis*, *Blastommycesdermatidisis* provided, comprising a compound according to the above aspect or embodiments of the invention.

Dosage forms may be for administration via various routes, including nasal, buccal, rectal, transdermal or oral administration, or as an inhalation formulation or suppository. Alternatively, dosage forms may be for parenteral administration, such as intravenous, intrahepatic, or especially subcutaneous, or intramuscular injection forms. Optionally, a pharmaceutically acceptable carrier and/or excipient may be present.

According to one aspect of the invention, a method for manufacture of a medicament for preventing or treating antifungal infection by the Ascomycota and Basidiomycota famlies in particular species of *Candida albicans*, *Candida glabrata*, *other Candidiasis*, *Aspergillus fumigatus, Aspergillus flavus*, *Cryptococcus neoformans, Cryptococcus gattii, Histoplasma capsulatum, Pneumocystis jirovecii, Tinea pedis, Tinea corporis, Blastommycesdermatidis* comprising the use of a compound according to the above aspect or embodiments of the invention. Medicaments according to the invention are manufactured by methods known in the art, especially by conventional mixing, coating, granulating, dissolving or lyophilizing. According to a fifth aspect of the invention, the compounds defined in the first aspect of the invention are provided as an inhibitor of lanosterol-14α-demethylase.

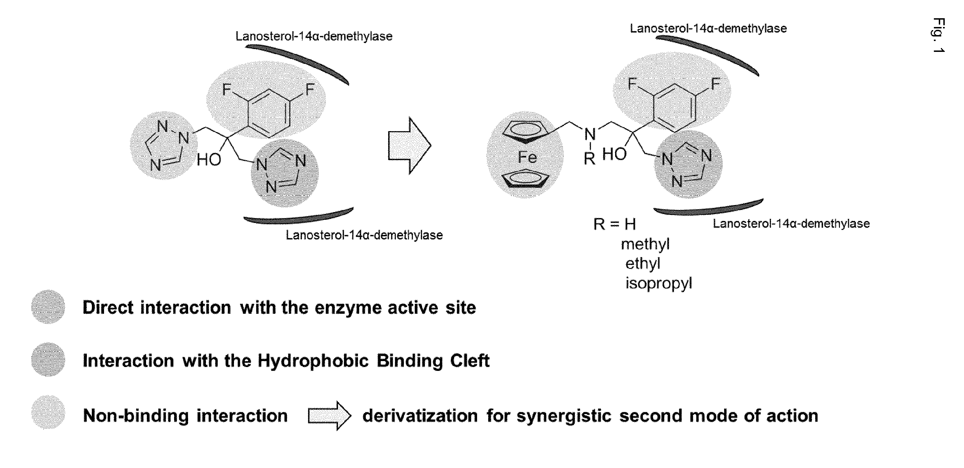

Lanosterol-14α-demethylase plays a central role in the biosynthesis of ergosterol, an important fungal wall constituent. The inhibition of this protein leads to wall instability and fungal death. Without wishing to be bound by theory the inventors believe that the compounds defined in the first aspect of the invention interact with Lanosterol-14α-demethylase. This interaction includes different parts of their chemical structure as indicated in figure 1. The interaction results in the inactivation of Lanosterol-14α-demethylase and thereby inhibits fungal growth.

Wherever reference is made herein to an embodiment of the invention, and such embodiment only refers to one feature of the invention, it is intended that such embodiment may be combined with any other embodiment referring to a different feature. For example, every embodiment that defines Y may be combined with every embodiment that defines Rₙ or X to characterize a group of compounds of the invention or a single compound of the invention with different properties.

The invention is further illustrated by the following examples and figures, from which further embodiments and advantages can be drawn. These examples are meant to illustrate the invention but not to limit its scope.

### Brief description of the figures

- Fig. 1: shows the strategy for drug design of the compound of the first aspect of the invention.
- Fig. 2: shows example of colony formation assay for the first generation of complexes. Compounds tested were: compound 4a (H), compound 5a (methyl), compound 6a (ethyl) and compound 7a (isopropyl). The compound concentration tested was declining from left to right. The numbers indicate EC₅₀-values in µM.

### Examples

### Synthesis:

The synthesis of the new antifungal drug candidates was designed in order to minimize the synthetic steps, to involve cheap reagents and to be performed under mild reaction conditions. Starting from the commercially available 2,4-difluoro-a-(1*H*-1,2,4-triazolyl)acetophenone (CAS number 86404-63-9) a Corey-Chaykovski epoxidation was performed (see Scheme 3). In parallel the *N*-substituted methylferrocenes were prepared starting from ferrocenylaldheyde upon imine formation and subsequent reduction in presence of NaBH₄. Finally, the epoxide ring opening via nucleophilic addition by the *N*-alkyl-methhylaminoferrocene yielded a series of the compounds of the present invention. Four different alkyl side chains, with R= -H, -methyl, -ethyl or -isopropyl, with increasing degree of hindrance and lipophilicity were envisaged in order to obtain a preliminary structure-activtiy relationship. All complexes were characterized by Nuclear Magnetic Resonance techniques, Mass Spectrometry, Infrared Spectroscopy and their purity assessed *via* elemental analysis.

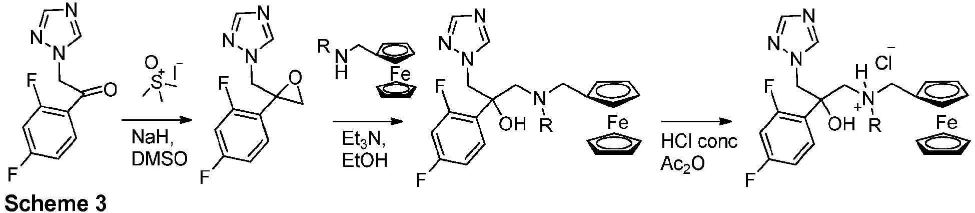

### Host cytotoxicity:

In both topic and systemic applications, antimycotic drugs are getting in contact with patient tissues. It is therefore of utmost importance that new drug candidates display a lack of host toxicity. Thus, the antiproliferative effect of the new complexes were investigated *in vitro* on human retinal pigment epithelial cell (RPE-1-hTert). As expected, fluconazole as a reference substance did not affect cell viability. The compounds of the present invention displayed all IC₅₀ values in the mid-high micromolar range, following the order of potency isopropyl> ethyl > methyl> H (see table 1). This suggested that a decrease in the size of the *N*-substituent correlates with a decrease in host toxicity.

**Table 1: Host cytotoxicity**

| **Compound** | **IC₅₀ [µM]** |
|---|---|
| **Fluconazole** | >100 |
| **H** | >100 |
| **Methyl** | 74.7 ^{±5.2} |
| **Ethyl** | 51.2 ^{±0.1} |
| **Isopropyl** | 48.4 ^{±2.9} |

### Antifungal activity:

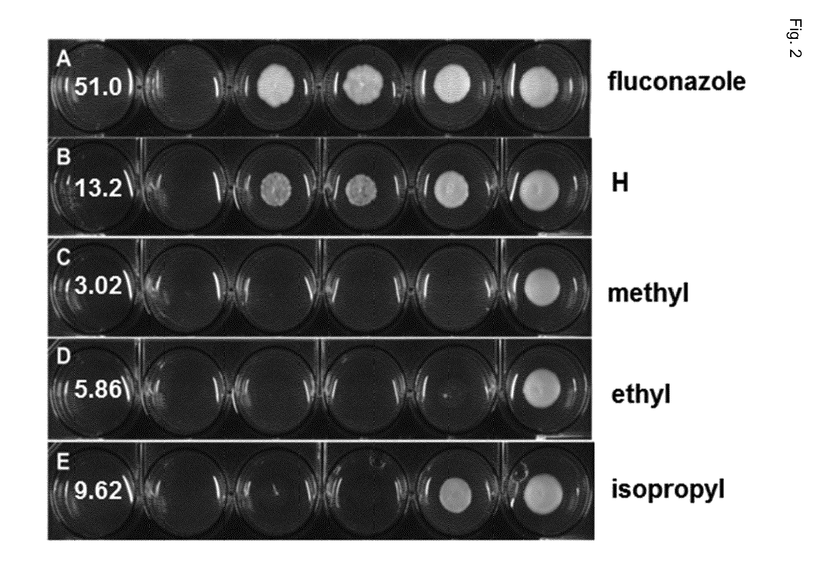

To evaluate the antifungal acitivty of the new drug candidates, a well-plate based assay was used. The first investigation of antimycotic activity has been performed on a well-known model, namely *Candida Robusta.* Wild type colony of C. *robusta* was cultured and diluted to start the growing phase just before treatment and then seeded at different concentrations on the agar terrain poured with different concentrations of the target complexes and incubated for 24 h at 30 °C. Interestingly, all complexes displayed a better antifungal activity in comparison with the parent drug (figure 2). Tertiary amines as linkers showed a better profile compared to secondary amines. A small substituent on the N resulted in higher mycotoxicity with R=-methyl resulting in the highest antimycotic activity.

### In vivo screening:

*In vivo* studies were performed on pathological isolated strains. The inventors chose the methyl derivative since it displayed the best *in vitro* mycotoxicity and a low cytotoxicity against human cells. The compounds of the present invention were tested against *Candida albicans*, its azole-resistant type and three different pathologic fungi, namely *Penicillumpaneum*, *Aspergillus glaucus* and *Trichosporonasahi* (see table 2). Of utmost interest, the new antifungal drug candidate displayed a very strong activity towards almost all investigated strains (only in the case of *A. glaucus* was the MIC₅₀ not in the nanomolar range). Moreover, the antimycotic potency of the complex overcomes the values of the parent drug up to a factor of 800 fold (e.g. *P.paneum*) and was able to overcome azole resistance (e.g. *C*. *albicans* resistant strain).

**Table 2: In vivo antifungal activity**

| **Strain** | **MethylMIC₅₀ [µM]** | **FluconazoleMI C₅₀ [µM]** |
|---|---|---|
| **C. albicans** | 0.07 | 0.8 |
| **C. albicans** | 0.7 | >209 |
| **(res. Strains)** | | |
| **P. paneum** | 0.25 | >209 |
| **A. glaucus** | >1 | 0.8 |
| **T. asahi** | 0.13 | 1.6 |

### Material and methods

### Materials:

All chemicals were of reagent grade quality or better, obtained from commercial suppliers and used without further purification. Solvents were used as received or dried over 4 A or 3 A molecular sieves. THF and Et₂O were freshly distilled under N₂ by employing standard procedures. All syntheses were carried out using standard Schlenk techniques. Instrumentation and methods: ¹H- and ¹³C-NMR spectra were recorded in deuterated solvents on a Bruker DRX 400 or AV2 500 at 30 °C. The chemical shifts δ, are reported in ppm. The residual solvent peaks have been used as internal reference. The abbreviations for the peak multiplicities are as follows: s (singlet), d (doublet), dd (doublet of doublet), t (triplet), q (quartet), m (multiplet) and br (broad). Infrared spectra were recorded on a PerkinElmer spectrum BX TF-IR spectrometer and KBrpresslings were used for solids. Signal intensities are abbreviated w (weak), m (medium), s (strong) and br (5 broad). ESI mass spectra were recorded on a Bruker Esquire 6000 or on a Bruker maxis QTOF-MS instrument (Bruker Daltonics GmbH, Bremen, Germany). LC-MS spectra were measured on an AcquityTM from Waters system equipped with a PDA detector and an auto sampler using an Agilent Zorbax 300SB-C18 analytical column (5.0 µm particle size, 100 A pore size, 150 × 3.0 mm) or an Macherey - Nagel 100 - 5 C18 (3.5 µm particle size, 300 A pore size, 150 × 3.0 mm). This LC was coupled to an Esquire HCT from Bruker (Bremen, Germany) for the MS measurements. The LC run (flow rate: 0.3 mL min-1) was performed with a linear gradient of A (distilled water containing 0.1% v/v formic acid) and B (acetonitrile, Sigma-Aldrich, HPLCgrade), t = 0 min, 5% B; t = 3 min, 5% B; t = 17 min, 100% B; t = 20 min, 100% B; t = 25 min, 15 5% B. High-resolution ESI mass spectra were recorded on a Bruker maxis QTOF-MS instrument (Bruker Daltonics GmbH, Bremen, Germany). The samples (around 0.5 mg) were dissolved in 0.5 mL of MeCN/H₂O 1:1 + 0.1% HCOOH. The solution was then diluted 10:1 and analysed via continuous flow injection at 3 µl.min-1. The mass spectrometer was operated in the positive electrospray ionization mode at 4000 V capillary voltage, -500 V 20 endplate offset, with a N2 nebulizer pressure of 0.4 bar and dry gas flow of 4.0 l/min at 180°C. MS acquisitions were performed in the full scan mode in the mass range from *m*/*z* 100 to 2000 at 20'000 resolution and 1 scan per second. Masses were calibrated with a 2 mmol/l solution of sodium formate over *m*/*z* 158 to 1450 mass range with an accuracy below 2 ppm.

### Yeast Culture

Wild type C. *robusta* was freshly inoculated and cultured in autoclaved YPD buffer containing 2% bacto peptone, 1% bacto yeast extract and 2% glucose anhydrous at 30°C (Kuehner shaker Labtherm, Kuehner Switzerland).

### Toxicity towards Fungi

The activity of the compounds of the present invention C. *robusta* was evaluated via a colony formation assay as described in R. Rubbiani, O. Blacque, G. Gasser, Dalton Trans. 2016, 45, 6619-6626. Briefly, one day before treatment, an aliquot of wild type C. *robusta*was inoculated in 5 mL YPD buffer solution (see above) and incubated overnight at 30°C. The fungal proliferation was then quantified at 600 nm in a Cary60 UV/Vis (Agilent Technologies). The SC was then seeded at different concentrations of the yeast (representing OD of 0.2, 0.02, 0.002 and 0.0002 for different colony growth rate) in 24-wells plates containing 2 mL growing terrain. The growing terrain was composed of YPD containing 2% of agar, autoclaved and kept at 50°C (water bath, Kotterman AG). Just before use, the growing terrain was treated with increasing concentrations of the compounds of the invention or with fluconazole (used for comparison) and poured into the different wells, before solidification. The treated plates were loaded with *C*. *robusta* and incubated for 24 h at 30 °C. The fungal colony formation was monitored with Alpha Digitec camera (Bucher Biotec) and the colony density was calculated with Alphalmager software (v1.3.0.7) with multiplex band analysis mode / single tool. A series of blanks (with the terrain not seeded with *C*. *robusta*), negative controls (with the terrain seeded with *C*. *robusta* and treated just DMSO as vehicle) and Clotrimazole as a positive control were performed.

### Cell Culture

Human fibroblast (MRC-5) and retinal pigment epithelial (RPE) cell lines were cultured in F-10 medium supplemented with 10% fetal calf serum (FCS, Gibco), 100 U/mL penicillin, 100 µg/mL streptomycin or DMEM medium (Gibco) supplemented with 10% fetal calf serum (FCS, Gibco), 100 U/mL penicillin, 100 µg/mL streptomycin, respectively, at 37 °C and 6% CO₂.

### Cytotoxicity studies

Cytotoxicity studies of the compounds of the present invention were performed by a fluorometric cell viability assay using resazurin (Promocell GmbH). Briefly, one day before treatment the cells were plated in triplicate in 96-well plates at a density of 4 × 10³ cells per well in 100 µL. Upon treating the cells with increasing concentrations of the compounds of the present invention, the cells were incubated for 48 h at 37 °C/6% CO₂, the medium was removed, and 100 µL of complete medium containing resazurin (0.2 mg mL⁻¹ final concentration) was added. After 4 h of incubation at 37 °C/6% CO₂, the fluorescence of the highly red fluorescent resorufin product was quantified at 590 nm emission with 540 nm excitation wavelength using a SpectraMax M5 Microplate Reader. The results were expressed as mean ± standard deviation error of independent experiments.

### In vivo Antifungal Activity on Pathological Isolated Strain

*Candida* strains were tested for their susceptibility to the azole (7 dilution series, ranging from 1 mM to 0.01 mM) following the European Committee for Antimicrobial Susceptibility Testing protocol (EUCAST Definitive Document EDef 7.2 Revision, 2012). Briefly, cells were grown in RPMI1640 medium supplied with 2.0% glucose, counted and inoculated at a concentration of 1-5 x 10⁵ CFU/ml. MIC₅₀ values were determined at 530 nm using a spectrophotometer, as the lowest concentration of the drug that resulted in ≥ 50% inhibition of growth relative to the growth control after 48 h incubation.

### Synthesis of the intermediates

### (R)/(S)-1-((2-(2,4-difluorophenyl)oxiran-2-yl)methyl)-1H-1,2,4-triazole:

In a 250 mL round-bottom flask, NaH (0.02 g, 0.79 mmol) was dispersed in dry DMSO (10 mL) and trimethylsulfoxonium iodide (0.17 g, 0.77 mmol) was added. The suspension was stirred at room temperature for 20 min, while the mixture became clear. 1-(2,4-difluorophenyl)-2-(1*H*-1,2,4-triazol-1-yl)ethan-1-one (0.14 g, 0.64 mmol) in dry DMSO (20 mL) was added and the reaction mixture was heated to 85 °C for 5 h. After cooling the solution to room temperature, the reaction mixture was poured into cold H₂O (80 mL, 10°C).

The product was extracted with EtOAc (2 x 50 mL) and the combined organic phases were washed with H₂O (50 mL), brine (50 mL), dried over MgSO₄, filtered and evaporated to give (*R*)/(*S*)-1-((2-(2,4-difluorophenyl)oxiran-2-yl)methyl)-1*H*-1,2,4-triazole as an orange oil, which was stored at -20 °C and used for the next reaction step within the next 16 h. Yield: 76% (0.12 g, 0.51 mmol). The spectroscopic data matched those known in the art.

### Ferrocenecarbaldehyde oxime:

In a 50 mL round-bottom flask, ferrocene carboxaldehyde (0.30 g, 1.40 mmol), NaOH (0.34 g, 8.5 mmol) and hydroxylamine hydrochloride (0.39 g, 5.61 mmol) were dissolved in EtOH (15 mL). The solution was refluxed for 3 h and then cooled to room temperature. After hydrolysis of the mixture (35 mL H₂O) the target complex was extracted with CH₂Cl₂ (3 x 50 mL). The combined organic phases were dried over MgSO₄, filtered and evaporated to afford ferrocenecarbaldehyde oxime as an orange amorphous solid. Yield: 81% (260 mg, 1.13 mmol). The spectroscopic data matched those known in the art.

### Ferrocenylmethanamine:

In a 50 mL round-bottom flask, LiAlH₄ (0.23 g, 6.06 mmol) was dissolved in THF (8 mL). A solution of crude ferrocenecarbaldehyde oxime (0.26 g, 1.14 mmol) in THF (8 mL) was added dropwise and the mixture was refluxed for 3 h. The reaction was quenched with H₂O (25 mL) and extracted with CH₂Cl₂ (3 x 50 mL). The combined organic layers were dried over MgSO₄, filtered and evaporated to give ferrocenylmethanamine as red oil. Yield: 89% (0.22 mg, 1.01 mmol). The spectroscopic data matched those known in the art.

### 1-Ferrocenyl-N-alkylmethanimine:

In a 25 mL round-bottom flask, ferrocene carboxaldehyde (0.25 g, 1.17 mmol) was dissolved in 40% aqueous alkylamine solution (55.45 mmol). After stirring at room temperature for 2 h, the product was extracted with Et₂O (2 x 30 mL). The combined organic phases were washed with H₂O (20 mL), dried over MgSO₄, filtered and evaporated to give 1-ferrocenyl-*N-*alkylmethanimine as a red crystalline solid or red oil, which was used for the next reaction step without further purification. Yield: 74% (0.25 g, 1.10 mmol). The spectroscopic data matched those known in the art.

### syn/anti-N-(ferrocenylmethyl)-2-(2,4-difluorophenyl)-2-hydroxy-3-(1H-1,2,4-triazol-1-yl)propan-1-aminium chloride:

In a 50 mL round-bottom flask, crude 1-((2-(2,4-difluorophenyl)oxiran-2-yl)methyl)-1H-1,2,4-triazole (0.12 g, 51 mmol) was dissolved in dry THF (10 mL). Ferrocenylmethanamine (0.09 g, 0.42 mmol) and Et₃N (0.11 mL, 0.79 mmol) were added to the solution. The reaction mixture was stirred at room temperature for 4 h and then at reflux for 7.5 h. The solvent were evaporated and the residue purified by column chromatography on silica using EtOAc:hexane (4:1) and EtOAc:CH₂Cl₂:MeOH (40:1:1) as the eluent (Rf = 0.62, EtOAc:CH2Cl2:MeOH (5:1:1)) to afford (R)/(S)-1-((ferrocenylmethyl)amino)-2-(2,4-difluorophenyl)-3-(1H-1,2,4-triazol-1-yl)propan-2-ol as an orange oil. The obtained oil (0.11 g, 0.24 mmol) was then dissolved acetone (4 mL) and 32% aq. HCl (26.5 µL, 0.27 mmol) was added. After 3 h of stirring, an orange precipitate was formed which was separated from the supernatant by centrifugation and decantation. The orange powder was washed with acetone, Et₂O, and dried on high vacuum to afford *syn*/*anti-N-*(*cyclopentylmethyl*)*-*2*-*(2,4-difluorophenyl)-2-hydroxy-3-(1*H*-1,2,4-triazol-1-yl)propan-1-aminium chloride as a yellow amorphous solid. Yield: 82% (0.10 g, 0.20 mmol). IR (cm-1): 3105*w*, 1615*m*, 1500*m*, 1395*w*, 1270*m*, 1140*s*, 970*m*, 850*s*, 780*w*, 675*s*, 445*s*. ¹H NMR (500 MHz, CD₃OD) : δ/ppm = 8.28 (s, 1H, NC*H*N), 7.86 (*s*, 1 H, NCHN), 7.56 - 7.51 (*m*, 1 H, arom. H), 7.05 - 6.98 (*m*, 2H, arom. H), 4.69 (*d*, 2*J* = 15.0 Hz, 1H, *CH₂*NN), 4.64 (*d*, 2*J* = 15.0 Hz, 1H, *CH₂*NN), 4.40 (s, 1H, C₅*H*₄), 4.33 (s, 1 H, C₅*H*₄), 4.31 (s, 2H, C₅*H*₄), 4.21 (s, 5H, C₅*H*₅), 4.02 (s, 2H, C*H*₂C₅H₄), 3.56 *(d, 2J* = 10.0 Hz, 1 H, NH₂C*H*₂COH), 3.33 (1 H, NH₂C*H*₂COH, peak covered by CD₃OD peak). ¹³C NMR (125 MHz, CD₃OD) : δ/ppm = 166.1, 166.0, 164.1, 164.0, 161.9, 161.8, 160.0, 159.9, 152.1, 146.4, 131.5, 131.4, 131.4, 131.4, 123.0, 123.0, 122.9, 122.9, 113.0, 113.0, 112.9, 112.8, 106.0, 105.8, 105.6, 76.3, 73.6, 73.6, 71.8, 71.0, 70.1, 56.5, 56.5, 52.4, 52.3, 1 peak covered by CD₃OD peak, but identified in ¹³C HSQC. ¹⁹F NMR (470 MHz, CD₃OD) : δ/ppm = 109.20, 110.88. ESI-MS: *m*/*z* (%) = 452.1 ([M-HCl]⁺, 100), 198.9 (C₁₁H₁₀Fe⁺, 25), 255.1 (C₁₁H₁₃F₂N₄O⁺, 20). Elemental Analysis (%): calcd. for C₂₂H₂₃N₄OF₂CIFe = C, 54.07; H, 4.74; N, 11.46. Found = C, 53.81; H, 4.71; N, 11.50.

### syn/anti-N-(ferrocenylmethyl)-2-(2,4-difluorophenyl)-2-hydroxy-N-methyl-3-(1H-1,2,4-triazol-1-yl)propan-1-aminium chloride:

In a 250 mL round-bottom flask, crude 1-((2-(2,4-difluorophenyl)oxiran-2-yl)methyl)-1*H*-1,2,4-triazole (0.18 g, 0.76 mmol) was dissolved together with 1-ferrocenyl-*N*-methylmethanamine (0.19 g, 0.83 mmol) and Et₃N (0.37 mL, 2.66 mmol) in dry EtOH (4 mL). The reaction mixture was refluxed for 7 h. The solvent was evaporated and the residue was purified by column chromatography on silica using EtOAc:hexane:MeOH (22:22:1) as the eluent (Rf = 0.38, EtOAc:hexane:MeOH (22:22.1)) to afford (*R*)/(*S*)-1-((ferrocenylmethyl)(methyl)amino)-2-(2,4-difluorophenyl)-3-(1*H*-1,2,4-triazol-1-yl)propan-2-ol as an orange oil). The obtained oil (0.14 g, 0.29 mmol) was then dissolved in acetone (4 mL) and 32% aq. HCl (26.5 µL, 0.31 mmol) was added. After 3 h of stirring an orange precipitate was formed which was separated from the supernatant by centrifugation and decantation. The residue was washed with acetone, Et₂O and dried on high vacuum to afford syn/anti-N-(ferrocenylmethyl)-2-(2,4-difluorophenyl)-2-hydroxy-N-methyl-3-(1H-1,2,4-triazol-1-yl)propan-1-aminium chloride as a yellow-orange amorphous solid. Yield: 82% (0.11 g, 0.22 mmol). IR (cm-1): 3105*w*, 2350*w*, 1615*m*, 1500*m*, 1425*m*, 1270*m*, 1210*w*, 1135*m*, 1020*m*, 965*m*, 845*s*, 675*m*, 485*s*. ¹H NMR (500 MHz, CD₃OD): δ/ppm = 8.56 (*s*, 1 H, NCHN), 8.52 (*s*, 1 H, NCHN), 8.04 (*s*, 1 H, NCHN), 8.02 (*s*, 1 H, NCHN), 7.63 - 7.58 (*m*, 1H, arom. H), 7.13 - 7.00 (*m*, 4H, arom. H), 4.77 - 4.67 (*m*, 3H, *CH₂*NN), 4.55 (*d*, 2*J* = 15.0 Hz, 1 H, *CH₂*NN), 4.45 (s, 1 H, C₅*H*₄), 4.37 - 4.30 (*m*, 5H, C₅*H*₄; 1 H, NHC*H*₂COH), 4.24 (s, 5H, C₅*H*₅; 1 H, C₅*H*₄), 4.19 - 4.13 (*m*, 5H, C₅*H*₅; 1 H, C₅*H*₄; 1 H, NHC*H*₂COH), 4.01 - 3.98 (*m*, 2H, NHC*H*₂C₅H₄; 1 H, NHC*H*₂COH) , 3.75 *(d, 2J* = 15.0, 1 H, C*H*₂C₅H₄), 3.55 *(d, 2J* = 15.0, 1H, C*H*₂C₅H₄), 3.47 *(d, 2J* = 15.0, 1H, NHC*H*₂COH), 2.87 (s, 3H, C*H*₃), 2.49 (s, 3H, C*H*₃). ¹³C NMR (125 MHz, CD₃OD): δ/ppm = 166.2, 166.2, 166.1, 166.1, 164.2, 164.2, 164.1, 164.1, 161.5, 161.5, 159.6, 159.6, 152.5, 146.5, 131.3, 131.3, 131.3, 130.8, 130.8, 130.8, 124.3, 124.3, 124.3, 123.4, 123.3, 123.3, 113.5, 113.5, 113.3, 113.2, 113.2, 113.1, 113.1, 106.5, 106.3, 106.3, 106.1, 106.1, 105.8, 105.8, 74.8, 74.6, 74.6, 74.1, 74.1, 73.2, 73.2, 72.9, 72.9, 72.3, 72.3, 71.8, 71.8, 71.5, 71.5, 71.4, 71.2, 70.2, 60.7, 60.5, 59.7, 59.6, 57.1, 56.8, 49.6, 49.5, 49.5, 49.3, 49.3, 49.2, 49.0, 48.8, 48.7, 48.5, 44.5, 43.2. ¹⁹F NMR (470 MHz, CD₃OD): δ/ppm = 107.75, 108.88, 110.23, 110.41. ESI-MS: *m*/*z* (%) = 467.1 ([M-Cl]⁺, 100), 198.9 (C₁₁H₁₀Fe⁺, 47), 269.1 (C₁₂H₁₄F₂N₄O⁺, 20). Elemental Analysis (%): calcd. for C₂₃H₂₅N₄OF₂CIFe •·H₂O = C, 53.05; H, 5.23; N, 10.76. Found = C, 52.87; H, 5.65; N, 10.33.

### syn/anti-N-(ferrocenylmethyl)-2-(2,4-difluorophenyl)-2-hydroxy-N-ethyl-3-(1H-1,2,4-triazol-1-yl)propan-1-aminium chloride:

In a 250 mL round-bottom flask, crude 1-((2-(2,4-difluorophenyl)oxiran-2-yl)methyl)-1*H*-1,2,4-triazole (0.18 g, 0.76 mmol) was dissolved together with 1-ferrocenyl-*N*-ethylmethanamine (0.17 g, 0.70 mmol) and Et₃N (0.37 mL, 2.66 mmol) in dry EtOH (4 mL). The reaction mixture was refluxed for 6 h. The solvent was evaporated and the residue was purified by column chromatography on silica using EtOAc:hexane:MeOH (22:22:1) as the eluent (Rf = 0.64, EtOAc:hexane:MeOH (22:22.1)) to afford (*R*)/(*S*)-1-((ferrocenylmethyl)(ethyl)amino)-2-(2,4-difluorophenyl)-3-(1*H*-1,2,4-triazol-1-yl)propan-2-ol as an orange oil). The obtained oil (0.12 g, 0.25 mmol) was dissolved in acetone (4 mL) and 32% aq. HCl (25.4 µL, 0.26 mmol) was added. After 3 h of stirring, an orange precipitate was formed which was separated from the supernatant by centrifugation and decantation. The orange powder was washed with acetone, Et₂O and dried on high vacuum to afford *syn*/*anti-N*-(ferrocenylmethyl)-2-(2,4-difluorophenyl)-2-hydroxy-*N*-ethyl-3-(1*H*-1,2,4-triazol-1-yl)propan-1-aminium chloride as a yellow amorphous solid. Yield: 93% (0.11 g, 0.21 mmol). IR (cm-1): 3040*w*, 1615*w*, 1500*m*, 1415*m*, 1255*w*, 1145*m*, 970*m*, 850*m*, 640*s*, 500*s*. ¹H NMR (500 MHz, CD₃OD) δ/ppm = 9.48 (*s*, 1H, NCHN), 9.46 (*s*, 1 H, NCHN), 8.56 (*s*, 1 H, NCHN), 8.55 (*s*, 1 H, NCHN), 7.65 - 7.59 (*m*, 2H, arom. H), 7.22 - 7.06 (*m*, 4H, arom. H), 5.03 - 4.93 (*m*, 3H, C*H*2NN), 4.70 *(d, 2J* = 15.0 Hz, 1 H, *CH₂*NN), 4.53 (*s*, 1 H, C₅*H*₄), 4.44 - 4.21 (*m*, 5H, C₅*H*₅; 7H, C₅*H*₄; 2H, NHC*H*₂COH), 4.09 (s, 5H, C₅*H*₅), 3.92 - 3.64 (*m*, 2H, NHC*H*₂COH; 4H, C*H*₂C₅H₄), 3.28 - 3.26 (*m*, 1 H, C*H*₂CH₃), 3.16 - 3.12 (*m*, 1 H, C*H*₂CH₃), 2.97 - 2.94 (*m*, 2H, C*H*₂CH₃), 1.33 (*t*, 3*J*= 7.5 Hz, 3H, C*H*₃), 1.11 (*t*, 3*J* = 7.5 Hz, 3H, C*H*₃).¹³C NMR (125 MHz, CD₃OD) : δ/ppm = 165.1, 164.9, 163.0, 162.9, 160.4, 160.3, 158.5, 158.3, 144.9, 143.4, 130.0, 129.7, 121.7, 112.1, 112.0, 105.3, 104.9, 104.7, 104.5, 73.2, 72.7, 72.0, 71.9, 71.7, 71.4, 70.7, 70.0, 70.0, 69.0, 68.8, 57.0, 56.3, 55.6, 55.4, 54.9, 54.7, 50.6, 50.2, 7.7, 7.5. ¹⁹F NMR (470 MHz, CD₃OD): δ/ppm = 107.51, 108.62, 109.80. ESI-MS: *m*/*z* (%) = 481.1 ([M-Cl]⁺, 100), 198.9 (C₁₁H₁₀Fe⁺, 43), 283.1 (C₁₃H_{17F2}N₄O⁺, 16). Elemental Analysis (%): calcd. for C₂₄H₂₇N₄OF₂ClFe • 2 H₂O = C, 52.14; H, 5.65; N, 10.13. Found = C, 52.00; H, 5.06; N, 9.90.

### syn/anti-N-(ferrocenylmethyl)-2-(2,4-difluorophenyl)-2-hydroxy-N-isopropyl-3-(1H-1,2,4-triazol-1-yl)propan-1-aminium chloride:

In a 250 mL round-bottom flask, crude 1-((2-(2,4-difluorophenyl)oxiran-2-yl)methyl)-1*H*-1,2,4-triazole (0.09 g, 0.38 mmol) was dissolved together with 1-ferrocenyl-*N-*isopropylmethanamine (0.24 g, 0.96 mmol) and Et₃N (0.32 mL, 2.48 mmol) in dry EtOH (5 mL). The reaction mixture was refluxed for 7.5 h. The solvent was evaporated and the residue was purified by column chromatography on silica using EtOAc:hexane:MeOH (22:22:1) as the eluent to afford (*R*)/(*S*)-1-((ferrocenylmethyl)(isopropyl)amino)-2-(2,4-difluorophenyl)-3-(1*H*-1,2,4-triazol-1-yl)propan-2-ol as an orange oil). The obtained oil (0.09 g, 0.18 mmol) was then dissolved in acetone (4 mL) and 32% aq. HCl (19.1 µL, 0.19 mmol) was added. After 3 h of stirring an orange precipitate formed which was separated from the supernatant by centrifugation and decantation. The orange powder was washed with acetone, Et₂O and dried on high vacuum to afford syn/anti-N-(ferrocenylmethyl)-2-(2,4-difluorophenyl)-2-hydroxy-N-isopropyl-3-(1 H-1,2,4-triazol-1-yl)propan-1-aminium chloride as a yellow amorphous solid. Yield: 77% (0.07 g, 0.13 mmol). IR (cm-1): 3010w, 2255w, 1615w, 1495m, 1395m, 1270m, 1140s, 1090m, 970m, 830w, 680m, 495s. ¹H NMR (500 MHz, CD₃OD δ/ppm = 8.39 (s, 1 H, NCHN), 8.30 (s, 1 H, NCHN), 7.96 (s, 2H, NCHN), 7.76 - 7.70 (m, 1 H, arom. H), 7.62 - 7.58 (m, 1 H, arom. H), 7.30 - 7.22 (m, 2H, arom. H), 7.20 - 7.06 (m, 2H, arom. H), 4.89 - 4.76 (m, 3H, CH₂NN), 4.62 (d, 2J = 10.0 Hz, 1 H, CH₂NN), 4.51 - 4.35 (m, 4H, C5H4), 4.30 - 4.23 (m, 5H, C₅H₅, 2H, C₅H₄), 4.17 (s, 1 H, C₅H₄) , 4.01 (s, 5H, C₅H₅, 1H, C₅H₄), 3.92 (d, 2J = 15.0 Hz, 1H, CH₂), 3.75 3.57 (m, 7H, CH₂), 1.33 - 1.06 (m, 12H, CH₃). ¹³C NMR (125 MHz, CD₃OD): δ/ppm = 166.3, 166.2, 166.1, 166.0, 164.4, 164.3, 164.2, 164.1, 162.1, 162.0, 161.8, 161.7, 160.1, 1560.0, 159.8, 159.7, 152.3, 146.8, 146.5, 131.6, 131.6, 131.6, 131.5, 131.1, 124.9, 124.3, 122.9, 122.9, 122.8, 113.5, 113.4, 113.1, 113.0, 112.8, 112.8, 106.5, 106.2, 106.0, 105.9, 105.9, 105.7, 105.5, 75.4, 74.5, 74.0, 73.7, 73.7, 73.2, 73.0, 72.9, 72.9, 72.6, 72.2, 71.4, 71.2, 71.2, 70.3, 70.1, 58.2, 56.5, 55.6, 54.9, 54.5, 53.9, 53.0, 50.8, 50.8, 24.2, 18.6, 18.5, 18.3, 16.7, 14.8. ¹⁹F NMR (470 MHz, CD₃OD) : δ/ppm = 107.53, 108.52, 109.90, 110.27. ESI-MS: m/z (%) = 495.1 ([M-Cl]⁺, 100), 198.9 (C₁₁H₁₀Fe⁺, 40), 297.2 (C₁₄H₁₉F₂N₄O⁺, 17). Elemental Analysis (%): calcd. for C₂₅H₂₉N₄OF₂CIFe = C, 56.57; H, 5.51; N, 10.55. Found = C, 56.43; H, 5.61; N, 10.61.

### Examples of further modifications of the compounds of the present invention

### Modification of the ligand:

Different organic groups are added to the ferrocene scaffold following standard procedures starting from commercially available ferrocenyl dibromide. The starting material is reacted with PhCH₂ONH₂ and subsequently alkylated (S. Dey et al., Organometallics, 2015, 34, 3039-3046) obtaining the monoaminomonobromo derivative or with CICOOEt in presence of n-butyl-lithium followed by hydrolysis to obtain the monocarboxylic acid mono bromo derivative. The final organic derivatization is obtained via Friedel-Crafts reaction with the acyl chloride of the target substituent or via nucleophilic substitution as depicted in scheme 4.

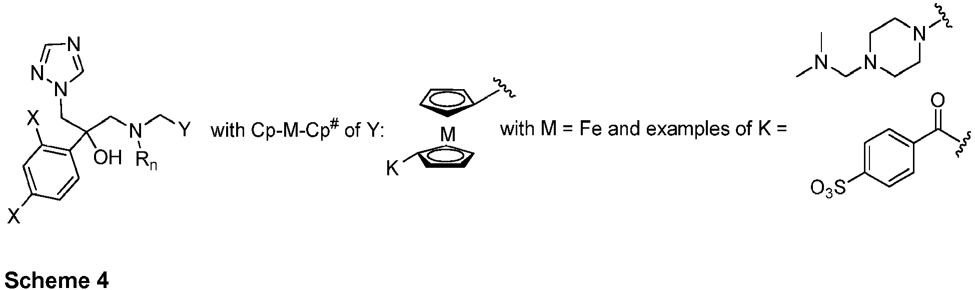

### Modification of the metallocenyl moiety

Different metallocenyls are synthesized following standard procedures. Cobaltoceneandruthenocene are commercially available and are halogenated in the presence of Br₂ and *tert*-butyl-lithium. The obtained bromo-metallocenes undergo a microwave-assisted nucleophilic substitution with NaN₃ (according to Y. Ju etal.,J. Org. Chem.,2006,71, 6697-6700), followed by hydrolysis, resulting in the respective metallocenylcarboxaladheyde (according to Patra et al. Chem. Commun., 2011, 47, 114444-11446). The obtained metallocenylcarboxaladheydeis then reacted with hydroxylamine or alkylamine, reduced with NaBH₄ in order to obtain the N-alkyl-methylaminometallocenyl derivatives as depicted in scheme 5.

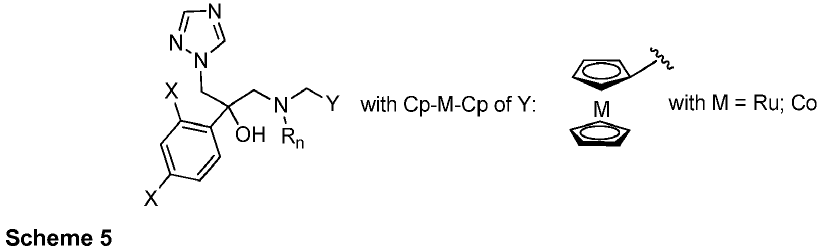

## Claims

1. A compound **characterized by** a general formula (1), 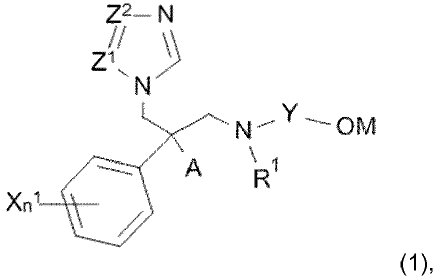 or a salt thereof,
wherein
- Z¹ and Z² are independently from each other -C or -N,
- A is selected from -H, -OH, -SH, a substituted or unsubstituted C₁ to C₄ thiol, a substituted or unsubstituted C₁ to C₄ alkylamine, a substituted or unsubstituted C₁₋C₄ alkoxy, a substituted or unsubstituted C₁₋C₄ aldehyde, a substituted or unsubstituted C₃-C₆ heterocycle,
- R¹ is hydrogen, a substituted or unsubstituted C₁ to C₅ alkyl, substituted or unsubstituted C₃-C₈ cycloalkyl, substituted or unsubstituted C₂ to C₅ alkenyl, substituted or unsubstituted C₂ to C₅ alkynyl,
- Y is a substituted or unsubstituted C₁ to C₅ alkyl, substituted or unsubstituted C₃ to C₈ cycloalkyl, substituted or unsubstituted C₂ to C₅ alkenyl, substituted or unsubstituted C₂ to C₅ alkynyl, -OR^{a}-, -C(O)R^{a}-, -C(S)OR^{a}-, -R^{a}-O-R^{a}-, -R^{a}-CO(=O)-_{R}^{a}-,
wherein each R^{a} is independently from each other R^{a} selected from the group consisting of hydrogen, unsubstituted C₁-C₄ alkyl, and C₁-C₄ alkyl substituted with C₁-C₄ alkoxy,
- OM is an organometallic compound independently selected from the group of an unsubstituted or substituted metal sandwich compound, an unsubstituted or substituted half metal sandwich compound or a metal carbonyl compound,
- n of Xₙ¹ is 1 or 2 and each X¹ independently from each other X¹ is -C(=O)OR², -C(=O)NR²₂, -C(=O)SR², -C(=S)OR², -C(NH)NR²₂, CN₄H₂, -NR²₂, -C(=O)R², -C(=S)R², -OR², -SR², -CF₃, -OCF₃, -SCF₃, -SOCF₃, -SO₂CF₃, -CN, -NO₂, -F, -Cl, -Br or -I, with each R² independently from any other R² being a hydrogen or C₁-C₄ alkyl.

2. The compound according to claim 1, wherein n of Xₙ¹ is 1 or 2, and each X¹ independently from any other X¹ is -CN, -CF₃, -OCF₃, -SCF₃, -SOCF₃, -SO₂CF₃,-F,-CI, -Br or -I, in particular each X¹ independently from any other X¹ is -F, -Cl, -Br or -I.

3. The compound according to any one of claims 1 or 2, wherein n of Xₙ¹ is 2 and one of the two X¹ is in ortho position and the other X¹ is in para position to the attachment position of the benzene moiety, or one of the two X¹ is in meta position and the other X¹ is in para position to the attachment position of the benzene moiety, in particular one of the two X¹ is in meta position and the other X¹ is in para position to the attachment position of the benzene moiety.

4. The compound according to any one of claims 1 to 3, wherein R¹ is hydrogen or a substituted or unsubstituted C₁ to C₃ alkyl.

5. The compound according to any one of claims 1 to 4, wherein Y is a substituted or unsubstituted C₁ to C₃ alkyl.

6. The compound according to any one of claims 1 to 5, wherein OM is an organometallic compound according to general formula (2a), 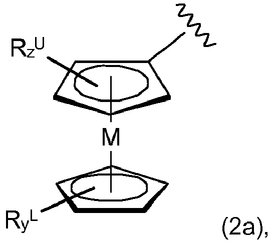
wherein M is a metal selected from Fe, Ru, Co, Ni, Cr, Os or Mn, and
z of R_{z}^{U} is 0, 1, 2, 3 or 4, and y of R_{y}^{L} is 0, 1, 2, 3, 4 or 5 and
each R^{L} and each R^{U} are independently from any other R^{L} and R^{U}selected from
- an unsubstituted or substituted C₁-C₁₀ alkyl, an unsubstituted or substituted C₂-C₁₀ alkenyl, an unsubstituted or substituted C₂-C₁₀ alkynyl, an unsubstituted or substituted C₃-C₈ cycloalkyl, an unsubstituted or substituted C₁-C₁₀ alkoxy, an unsubstituted or substituted C₃-C₈ cycloalkoxy,
- an unsubstituted or substituted C₆-C₁₄ aryl,
- an unsubstituted or substituted 5- to 10-membered heteroaryl, wherein 1 to 4 ring atoms are independently selected from nitrogen, oxygen or sulfur,
- an unsubstituted or substituted 5- to 10-membered heteroalicyclic ring, wherein 1 to 3 ring atoms are independently nitrogen, oxygen or sulfur,
- -OR³, -SR³, -C(O)R³, -C(S)R³, -C(O)OR³, -C(S)OR³, -C(O)SR³, -C(O)NR³R⁴, -NR³R⁴, -S(O)₂R³, -S(O)₂OR³ and -S(O)₂NR³R⁴,
wherein
R³and R⁴ are independently selected from the group consisting of hydrogen, unsubstituted C₁₋C₄ alkyl, and C₁₋C₄ alkyl substituted with C₁-C₄ alkoxy,
- general formula (3a) or (3b)

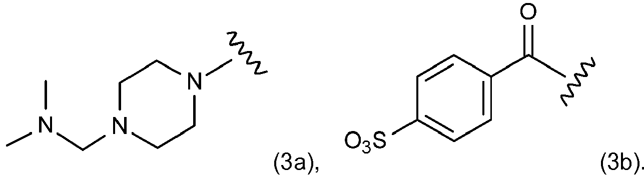

7. The compound according to claim 6, wherein each R^{L} and each R^{U} are independently from any other R^{L} and R^{U} selected from:
- -OR³, -SR³,-C(O)R³,-C(S)R³, -C(O)OR³, -C(S)OR³, -C(O)SR³, -C(O)NR³R⁴, -NR³R⁴, -S(O)₂ R³, -S(O)20 R³, and -S(O)₂NR³R⁴, wherein
R³ and R⁴ are independently selected from the group consisting of hydrogen, unsubstituted C₁-C₄ alkyl, and C₁-C₄ alkyl substituted with C₁-C₄ alkoxy,
or
- general formula (3a) or (3b)

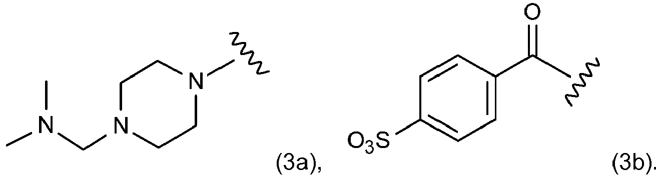

8. The compound according to any one of claims 6 or 7, wherein M is selected from the group of Fe, Ru or Co, wherein in particular M is Fe; and/or wherein y and z are 0.

9. The compound according to any one of claims 1 to 5, wherein OM is an organometallic compound according to the general formula (2b), 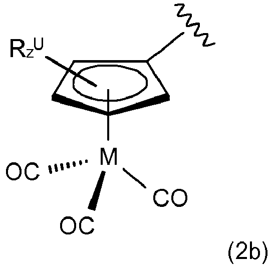
wherein M is a metal selected from the group of Mn, Re or Tc, and
z of R_{z}^{U} is 0, 1, 2, 3 or 4, and
each R^{U} is independently from any other R^{U} selected from
- an unsubstituted or substituted C₁-C₁₀ alkyl, an unsubstituted or substituted C₂-C₁₀ alkenyl, an unsubstituted or substituted C₂-C₁₀ alkynyl, an unsubstituted or substituted C₃-C₈ cycloalkyl, an unsubstituted or substituted C₁-C₁₀ alkoxy, an unsubstituted or substituted C₃-C₈ cycloalkoxy,
- an unsubstituted or substituted C₆-C₁₄ aryl,
- an unsubstituted or substituted 5- to 10-membered heteroaryl, wherein 1 to 4 ring atoms are independently selected from nitrogen, oxygen or sulfur,
- an unsubstituted or substituted 5- to 10-membered heteroalicyclic ring, wherein 1 to 3 ring atoms are independently nitrogen, oxygen or sulfur,
- -OR³, -SR³, -C(O)R³, -C(S)R³, -C(O)OR³, -C(S)OR³, -C(O)SR³, -C(O)NR³R⁴, -NR³R⁴, -S(O)₂R³, -S(O)₂OR³ and -S(O)₂NR³R⁴,
wherein
R³ and R⁴ are independently selected from the group consisting of hydrogen, unsubstituted C₁-C₄ alkyl, and C₁-C₄ alkyl substituted with C₁-C₄ alkoxy.

10. The compound according to any one of claims 1 to 5, wherein OM is an organometallic compound according to the general formula (2c), 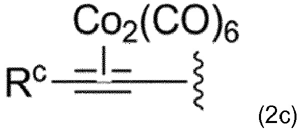
wherein R^{c} is selected from
- hydrogen,
- an unsubstituted or substituted C₁-C₁₀ alkyl, an unsubstituted or substituted C₂-C₁₀ alkenyl, an unsubstituted or substituted C₂-C₁₀ alkynyl, an unsubstituted or substituted C₃-C₈ cycloalkyl, an unsubstituted or substituted C₁-C₁₀ alkoxy, an unsubstituted or substituted C₃-C₈ cycloalkoxy,
- an unsubstituted or substituted C₆-C₁₄ aryl,
- an unsubstituted or substituted 5- to 10-membered heteroaryl, wherein 1 to 4 ring atoms are independently selected from nitrogen, oxygen or sulfur,
- an unsubstituted or substituted 5- to 10-membered heteroalicyclic ring, wherein 1 to 3 ring atoms are independently nitrogen, oxygen or sulfur, or
- -OR³, -SR³, -C(O)R³, -C(S)R³, -C(O)OR³, -C(S)OR³, -C(O)SR³, -C(O)NR³R⁴, -NR³R⁴, -S(O)₂R³, -S(O)₂OR³ and -S(O)₂NR³R⁴,
wherein
R³ and R⁴ are independently selected from the group consisting of hydrogen, unsubstituted C₁-C₄ alkyl, and C₁-C₄ alkyl substituted with C₁-C₄ alkoxy.

11. A compound according to any one of the claims 1 to 10 for use as a medicament.

12. A compound according to any one of the claims 1 to 10 for use as an antimycotic.

13. A compound according to any one of the claims 1 to 10 for use in the treatment of fungal infections caused by azole-resistant Candida albicans.

14. An inhibitor of lanosterol-14α-demethylase comprising the compound according to any one of claims 1 to 10.
